# EUROPEAN PATENT SPECIFICATION

(11) **EP 0 428 693 B1**
(45) Date of publication and mention of the grant of the patent: **24.07.1996**
(21) Application number: 90909245.4
(22) Date of filing: 31.05.1990
(51) Int. Cl.: C12Q 1/68, C07H 21/04

(54) **Nucleic acid probes for the detection of Chlamydia trachomatis and Chlamydia psittaci**
Nukleinsäuresonden zum Nachweis von Chlamydia trachomatis und Chlamydia psittaci
Sondes d'acides nucléiques pour la détection de Chlamydia trachomatis et de Chlamydia psittaci

(30) Priority: 31.05.1989 US 359293
(43) Date of publication of application: 29.05.1991
(60) Divisional application: 95203160.7
(73) Proprietor: AMOCO CORPORATION, Chicago Illinois 60680-0703 (US)
(72) Inventor: SHAH, Jyotsna, Nashua, NH 03060 (US); BUHARIN, Amelia, Framingham, MA 01701 (US); WILLIAMS, Charlotte, Northborough, MA 01532 (US); MAHAN, Donald, Grafton, MA 01519 (US); LANE, David, J., Milford, MA 01757 (US); KING, Walter, Maynard, MA 01754 (US)
(74) Representative: Sheard, Andrew Gregory
(86) International application number: US9002989
(87) International publication number: WO9015159

(56) References cited:
- EP-A- 0 272 009
- EP-A- 0 281 390
- EP-A- 0 305 145
- EP-A- 0 309 230
- EP-A- 0 339 783

## Description

### Field of the Invention

This invention relates to detecting bacteria belonging to the species Chlamydia trachomatis or Chlamydia psittaci and more specifically provides nucleic acid probes and compositions along with methods for their use for the specific detection of Chlamydia trachomatis or Chlamydia psittaci.

### Background of the Invention

The term "Chlamydia trachomatis" as used herein, refers to bacteria classified as such in Bergey's Manual of Systematic Bacteriology (P. H. A. Sneath, ed., 1986, PP. 729-736, Williams & Wilkins). Chlamydia trachomatis is a member of the genus Chlamydia, which has only one other member, Chlamydia psittaci. Chlamydia are a unique group of intracellular parasites. Chlamydia has a complex life cycle that is characterized by two forms, the elementary body (EB) which like a virus is infectious but not metabolically active, and the reticulate body (RB) which is metabolically active, not infectious and divides by binary fission like bacteria. The RB eventually matures into the EB which is released by the infected cell, a process which take about 2 days.

Because of their role in ocular, respiratory and sexually transmitted diseases, these organisms are a major health risk through the world (Nichols, R. L. and Manire, G. P.: Chlamydiae, in Microbiology 3rd Ed, Davis, B. D., Dulbecco, R., Eisen, H. N. and Ginsburg, H. S. {ed}. 1980, 776-784, Harper & Rov). C. trachomatis is responsible for some 350 million cases of trachoma in the developing world (Dawson, C. R., Proceedings of the Fifth International Symposium on Human Chlamydial Infections. Lund, Sweden, June 15-19, 1982, Mardh, P. A., Holmes, K. K., Oril, J. D., Piot, P., Schachter, J., [ed], 1982, 71-81, Elsevier Biomedical Press, Amsterdam, New York, Oxford). This is particularly tragic since trachoma is also the leading cause of preventable blindness (Jones, B. R., 1975, Trans. Opthalmol. Soc. UK, 95: 16-33). Infection by this organism is now recognized as the most common sexually transmitted disease in the industrialized world (Centers for Disease Control, Division of sexually transmitted disease. Chlamydia trachomatis infections. Policy guidelines for prevention and control. MMVK 1985; 34: 53S-74S), accounting for more than 4 million cases in the United States alone (Judson FN. 1985, J.Reprod. Med. 30: 269-272), Direct and indirect costs run into billions of dollars.

Among the more serious complications of infection are ectopic pregnancy and tubal infertility (Washington et al., 1987, JAMA 257: 2070-2072). These two manifestations of C. trachomatis infection reflect two further groupings: the trachoma biovar which causes ocular and genital infections and the lymphogranuloma veretrum (LGV) biovar. These two biovars are further subdivided in 15 serotypes: A, B, Ba, C (usually associated with follicular conjunctivitis), D through K, and L1 through L3.

Diagnosis of the disease is often lengthy and labor intensive. Cell culture is considered the "gold standard" for chlamydial diagnostics. Because of the time-consuming and tedious nature of cell culture, and the difficulty of growing many clinical isolates in tissue culture, a number of rapid laboratory methods have recently become available (see review by Barnes RC, 1989, Clin. Microbiol. Rev. 2: 119-136). The vast majority are antibody-based tests. While in certain instances these rapid assays have proven to be more sensitive or specific than cell culture, their utility lies in their rapidity.

It is an aspect of the present invention to provide nucleic acid probes which are specific for Chlamydia, which do not react with other bacteria or fungi which may be present in sampled materials, and which may be used in a variety of assay systems.

It is another aspect of the present invention to provide probes which can hybridize to target regions which can be rendered accessible to probes under normal assay conditions.

It is yet another aspect of the present invention to provide assays which avoid many of the disadvantages associated with traditional, multi-day culturing techniques.

While Kohne et al. (Biophysical Journal 8:1104-1118, 1968) discuss one method for preparing probes to rRNA sequences, they do not provide the teaching necessary to make Chlamydia trachomatis specific probes.

Pace and Campbell (Journal of Bacteriology 107:543-547, 1971) discuss the homology of ribosomal ribonucleic acids from diverse bacterial species and a hybridization method for quantitating such homology levels. Similarly, Sogin, Sogin and Woese (Journal of Molecular Evolution 1:173-184, 1972) discuss the theoretical and practical aspects of using primary structural characterization of different ribosomal RNA molecules for evaluating phylogenetic relationships. Fox, Pechman and Woese (International Journal of Systematic Bacteriology 27:44-57, 1977) discuss the comparative cataloging of 16S ribosomal RNAs as an approach to prokaryotic systematics. These references, however, fail to relieve the deficiency of Kohne's teaching with respect to Chlamydia trachomatis and in particular, do not provide Chlamydia trachomatis specific probes useful in assays for detecting Chlamydia trachomatis in clinical and other samples.

EP-A-0272009 does disclose probes which will hybridise to *Chlamydia trachomatis.* These probes are derived from selected regions of the 16S and 23S rRNA of *Chlamydia trachomatis.*

Ribosomes are of profound importance to all organisms because they serve as the only known means of translating genetic information into cellular proteins, the main structural and catalytic elements of life. A clear manifestation of this importance is the observation that all cells have ribosomes.

Ribosomes contain three distinct RNA molecules which, at least in Escherichia coli, are referred to as 5S, 16S and 23S rRNAs. These names historically are related to the size of the RNA molecules, as determined by their sedimentation rate. In actuality, however, ribosomal RNA molecules vary substantially in size between organisms. Nonetheless, 5S, 16S, and 23S rRNA are commonly used as generic names for the homologous RNA molecules in any bacteria, and this convention will be continued herein.

As used herein, probe(s) refer to synthetic or biologically produced nucleic acids (DNA or RNA) which, by design or selection, contain specific nucleotide sequences that allow them to hybridize under defined predetermined stringencies, specifically (i.e., preferentially, see below - Hybridization) to target nucleic acid sequences. In addition to their hybridization properties, probes also may contain certain constituents that pertain to their proper or optimal functioning under particular assay conditions. For example, probes may be modified to carry dete:tion ligands (e.g. fluorescien, 32-P, biotin, etc.), or to facilitate their capture onto a solid support (e. g., poly-deoxyadenosine "tails"). Such modifications are elaborations on the basic probe function which is its ability to usefully discriminate between target and non-target organisms in a hybridization assay.

Hybridization traditionally is understood as the process by which, under predetermined reaction conditions, two partially or completely complementary strands of nucleic acid are allowed to come together in an antiparallel fashion to form a double-stranded nucleic acid with specific and stable hydrogen bonds.

The stringency of a particular set of hybridization conditions is defined by the length and base composition of the probe/target duplex, as well as by the level and geometry of mispairing between the two nucleic acids.

Stringency may also be governed by such reaction parameters as the concentration and type of ionic species present in the hybridization solution, the types and concentrations of denaturing agents present, and/or the temperature of hybridization. Generally, as hybridization conditions become more stringent, longer probes are preferred if stable hybrids are to be formed. As a corollary, the stringency of the conditions under which a hybridization is to take place (e. g., based on the type of assay to be performed) will dictate certain characteristics of the preferred probes to be employed. Such relationships are well understood and can be readily manipulated by those skilled in the art.

As a general matter, dependent upon probe length, such persons understand stringent conditions to mean approximately 35°C-65°C in a salt solution of approximately 0.9 molar.

### Summary of the Invention

In accordance with the various principles and aspects of the present invention, there are provided nucleic acid probes complementary or homologous to at least 90% of a sequence comprising any ten consecutive nucleotides of the region 179 to 198 of the 16S sRNA of either Chlamydia trachomatis or Chlamydia psittaci (using the E. coli numbering system). The said probes which hybridize, under specific conditions, to the ribosomal RNA molecules (rRNA) or rRNA genes (rDNA) of Chlamydia trachomatis or Chlamydia psittaci do not hybridize, under the same conditions, to the rRNA or rDNA of other related bacteria which may be present in test samples. Therefore the probe(s) of the present invention provide the basis for development of a valuable nucleic acid hybridization assay for the specific detection of Chlamydia trachomatis or Chlamydia psittaci in clinical samples.

In our experience such nucleic acid hybridization based assays have been discovered to impart enhanced performance capabilities with respect to most currently available, microbiological methods for detection of bacteria in test samples, generally including:
a) increased sensitivity; i. e., the ability to detect fewer said bacteria in a given sample;
b) potentially significant reductions in assay cost due to the use of inexpensive reagents and reduced labor;
c) accurate identification of even biochemically unusual strains of the target bacteria;
d) faster results because such tests do not require the isolation of the target bacterium from the sample prior to testing.

It has been discovered that other advantages incurred by directing the probes of the present invention against rRNA include the fact that the rRNAs detected constitute a significant component of cellular mass. Although estimates of cellular ribosome content vary, actively growing E. coli may contain upwards of 50,000 ribosomes per cell, and therefore 50,000 copies of each of the rRNAs (present in a 1:1:1 stoichiometry in ribosomes). Chlamydia trachomatis elementary bodies are known to contain far fewer ribosomes (hundreds). Nevertheless, this number is still larger than most other potential cellular target molecules such as genes or RNA transcripts thereof, which are less ideal since they are present in much lower abundance.

A further unexpected advantage is that the rRNAs (and the genes specifying them) appear not to be subject to lateral transfer between contemporary organisms. Thus, the rRNA primary structure provides an organism-specific molecular target, rather than a gene-specific target as would likely be the case, for example of a plasmid-borne gene or product thereof which may be subject to lateral transmission between contemporary organisms.

Additionally, the present invention provides probes to Chlamydia trachomatis rRNA target sequences which are sufficiently similar in all Chlamydia trachomatis strains tested that they can hybridize to the target region in all such Chlamydia trachomatis. Advantageously, these same rRNA target sequences are sufficiently different in most non-*Chlamydia trachomatis* rRNAs that, under conditions where probe 781 hybridizes to *Chlamydia trachomatis* rRNAs, it does not hybridize to most non*-Chlamydia trachomatis* rRNAs. These probe characteristics are defined as inclusivity and exclusivity, respectively.

Probe 782 is the only probe which hybridizes to *Chlamydia psittaci* and not to *Chlamydia trachomatis.*

The discovery that probes could be generated with the extraordinary inclusivity and exclusivity characteristics of those of the present invention with respect to Chlamydia trachomatis was unpredictable and unexpected.

Further understanding of the principles and aspects of the present invention may be made by reference tc the tables wherein:
Table 1 - Shovs the nucleotide sequence of the preferred 16S rRNA-targeted probe 781 of the present invention aligned upon its Chlamydial target nucleotide sequences. The corresponding portions of the 16S rRNA from E. coli is shown for comparison. RNA (target) sequences are written 5' to 3', probe sequence is DNA and written 3' to 5'. Probe is shown along with the "core" region of variation upon which its inclusivity and exclusivity behavior are based.
Table 2 - Exemplifies the inclusivity behavior of the preferred 16S rRNA probe 781 toward a representative sampling of Chlamydia trachomatis serovars in a liquid-hybridization assay.
Table 3 - Exemplifies the exclusivity behavior of the preferred 16S rRNA probe 781 toward a representative sampling of non-Chlamydia trachomatis bacteria in a dot blot hybridization assay.

### Detailed Description of the Invention and Best Mode

### Probe Development Strategy.

The first step taken in the development of the probes of the present invention involved identification of regions of 16S and 23S rRNA which potentially could serve as target sites for Chlamydia trachomatis specific nucleic acid probes. As a practical matter, it is difficult to predict, a priori, which non-Chlamydia trachomatis organisms might be present in any test sample.

Because of the large number of such potential non-Chlamydia trachomatis bacteria, demonstrating exclusivity for any given probe sequence is not only unpredictable but also extremely difficult and laborious. A more rigorous criterion was adopted to obviate the need to know what non-Chlamydia trachomatis bacteria might be present in all test samples that ultimately will be screened using the probes.

This entailed knowledge of the phylogenetic relationships among Chlamydia trachomatis and between Chlamydia trachomatis and other groups of bacteria.

Specifically, an operating but previously unproven hypothesis was adopted that the exclusivity criterion could be satisfied by determining that if a particular target region in Chlamydia trachomatis rRNA could be identified which was sufficiently different from the homologous region in the rRNA of representative yet close evolutionary relatives of Chlamydia trachomatis, then a probe to such a sequence could be used to distinguish between Chlamydia trachomatis and the relatives by hybridization assay. Based on phylogenetic observations, it then was extrapolated that rRNA sequences of more distantly related organisms, even though their actual identity may not necessarily be known, should be predictably different in a particular region of sequence than the aforementioned close evolutionary relative of Chlamydia trachomatis. However, it cannot be predicted, a priori, whether such regions exist or if they do, where within the rRNA such regions will be located.

As the first step in identifying regions of Chlamydia trachomatis rRNA which could potentially serve as useful target sites for nucleic acid hybridization probes, complete nucleotide sequences of the 16S rRNAs from Chlamydia trachomatis was determined.

The nucleotide sequences were determined by standard laboratory protocols either by cloning (Maniatis et al., 1982, Molecular Cloning; A Laboratory Manual, Cold Spring Harbor Laboratory, New York, pp 545) and sequencing (Maxam and Gilbert, 1977, Proceedings of the National Academy of Science, USA 74:560-564: Sanger et al., 1977, Proceedings of the National Academy of Science, USA 74:5463-5467) the genes which specify the rRNAs, and/or by direct sequencing of the rRNAs themselves using reverse transcriptase (Lane et al., 1985, Proceedings of the National Academy of Science, USA 82:6955-6959).

The determined Chlamydia trachomatis rRNA nucleotide sequences were compared to other available rRNA nucleotide sequences, in particular to Chlamydia psittaci, which also was determined as part of this work.

Comparison of the sequences of Chlamydia trachomatis and its close relative Chlamydia psittaci proved especially valuable. A region of sequence was identified which appeared to be different in the two species of Chlamydia and between Chlamydia trachomatis and non-Chlamydia bacteria. The location of this region within the 16S rRNA sequences is shown in Table 1.

Oligonucleotide probes, 28 - 36 nucleotides in length, were designed which would hybridize preferentially to Chlamydia trachomatis, Chlamydia psittaci or both. These were designed : 1) to maximally utilize the nucleotide sequence differences useful for distinguishing Chlamydia trachomatis from Chlamydia psittaci or Chlamydia from other bacteria (indicated as upper case letters in the region of Core Variation, table 1) and, 2) to minimize the effect of self complementarity both locally within the target rRNA and between probe molecules. Optimizing these parameters as well as others discussed above (Background) results in probes of preferred specificity and hybridization efficiency.

Table 2 exemplifies the inclusivity behavior of the preferred probe 781 toward a representative sampling of Chlamydia trachomatis and a few non-Chlamydia trachomatis bacteria in a liquid hybridization assay.

Table 3 exemplifies the exclusivity behavior of the preferred probe 781 toward a representative sampling of non-Chlamydia trachomatis bacteria in a dot-blot hybridization assay.

### Physical Description of the Probes.

The foregoing probe selection strategy yielded a number of probes useful for identifying Chlamydia trachomatis or Chlamydia psittaci bacteria in samples. Probes 781 and 782 are designed from 16S rRNA sequences. The following preferred oligonucleotide probes are disclosed herein:

### 16S rRNA-Targeted Probes:

SEQ ID NO 1
Probe 781: 5'-CTTTAACGTTACTCGGATGCCCAAATATCGCCACAT-3' 36 bases

SEQ ID NO 2
Probe 782: 5'-CTTTAATATGTTTTAGATGCCTAAACATACCACAT-3' 35 bases

Probe 781 and its target sequence in the 16S of Chlamydia trachomatis and Chlamydia psittaci are shown in Table 1. The corresponding nucleotide positions of the E. coli 16S rRNAs also are shown. Since the E. coli sequences were among the first full 16S sequences obtained, the assigned position numbers are a convenient standard for explicitly identifying the homologous regions in the Chlamydia rRNAs under consideration.

More than one probe has been designed to a number of the target regions shown in Table 1 corresponding variously to complements of the sequences of Chlamydia trachomatis serovars LCV or K, or Chlamydia psittaci, or all three. The particular sequence upon which each probe is based (i.e., is complementary to) is provided in Table 1 by inspection of the aligned probe and target sequences.

Likewise, probes 781 and 782 are based on Chlamydia trachomatis (LGV is identical to K in this region) and Chlamydia psittaci 16S rRNA sequences, respectively.

The specific hybridization behaviors of the probes described above are dependent to a significant extent on the assay format in which they are employed. Conversely, the assay format will dictate certain of the optimal design features of particular probes. The "essence" of the probes of the invention is not to be construed as restricted to the specific string of nucleotides in the probed named above. For example, the length of these particular oligonucleotides was optimized for use in the dot blot assay (and certain other anticipated assays) described below. It is well known to one skilled in the art that optimal probe length will be a function of the stringency of the hybridization conditions chosen and hence the length of the instant probes may be altered in accordance therewith. Also, in considering sets comprised of more than one probe, it is desirable that all probes behave in a compatible manner in any particular format in which they are both employed. Thus, the exact length of a particular probe will to a certain extent reflect its specific intended use.

The "essence" of the probes described herein resides in the discovery and utilization of the Chlamydia trachomatis specific sequences described above and given in Table 1 (Core Variation).

### Hybridization Analysis of Probe Behavior.

The sequence data in Table 1 suggest that the probes of the present invention should exhibit a variety of useful hybridization properties with respect to the specific detection of Chlamydia trachomatis, Chlamydia psittaci or both to the exclusion of other bacteria. However, relatively few Chlamydia trachomatis and Chlamydia psittaci sequences were inspected. It is possible that sequence variation might exist in other Chlamydia trachomatis serotypes not inspected by sequence analysis. Such variation might reduce or eliminate hybridization by the prospective probes to some or many untested Chlamydia trachomatis serotypes.

Equally as important as the inclusivity behavior of the probes, is their exclusivity behavior, i.e., their reactivity toward non-Chlamydia bacteria. The number and types of non-Chlamydia strains which might be encountered in a potentially Chlamydia containing test sample are extremely large.

Therefore, the behavior of the probes toward representative Chlamydia trachomatis and non-Chlamydia trachomatis bacteria was determined by hybridization analysis using a liquid hybridization and a dot blot procedures.

While hybridization data for each of the individual probes of the present invention are provided, it should be noted that useful combinations (sets) of probes which exhibit hybridization behaviors that are the sum of the individual probes also is explicitly predicted by the data.

Example 1: Inclusivity Analysis of probe hybridization behaviour in liquid-hybridization assay.

The probes of the present invention or derivatives thereof could potentially be of significant value in a variety of hybridization formats. One such format, a dual probe, sandwich-type hybridization assay formats (e.g. the homopolymer capture, dual probe, liquid hybridization format described in USSN 277, 579; USSN 169,646, or USSN 233,683, equivalent to US-A-5147778, US-A-5370992 and US-A-5084565, respectively), is used in this example. In general in such an application, an oligonucleotide probe is modified at its 3' terminus to contain a tract ot deoxyadenosine (dA) residues ca. 20 - 200 residues long. This would be used to "capture" the target rRNA (following liquid hybridization) from the test sample onto a solid support (e.g., beads, plastic surface, filter, etc.) which had been suitably derivatized with poly-deoxythymidine (dT) for this purpose. A second probe is used as a "detection" probe and would be derivatized by some detectable ligand (e.g. 32-P, fluorescien, biotin, etc.). In principle, the detection probe could be an oligonucleotide or a longer DNA or RNA probe. Detection of the presence of the target nucleic acid in a test sample then is indicated by the capture of the detection ligand onto the solid surface through the series of hybridization interactions:

SOLID | \ (TARGET NUCLEIC ACID) /
|TTTTTTTTn \________________________________/
SUPPORT | |||||| ||||||||||||| |||||||||||||||
nAAAAAAAAA(Capture Probe) (Detection Probe)
| |
Ligand Ligand

This could occur only if the target nucleic acid is present in the test sample.

In this example, each oligonucleotide individually is used as a capture probe (for this purpose ca. 200-500 dA residues were appended to their 3' termini using terminal deoxynucleotidyl transferase). P-32 labeled "riboprobes," specific for 16S rRNA, were prepared as described below and used as "generic" detection probes.

Both riboprobes were prepared by transcription from plasmid vectors containing portions of the E. coli (16S rRNA) genes. The 16S riboprobe is an antisense transcript, ca. 567 nucleotides long, generated from a HindIII subfragment of the E. coli 16S rRNA gene (E. coli positions 1 to 567). Radioactive phosphorous 32 is incorporated into the riboprobes during the transcription reactions by using P-32 substituted nucleotide triphosphate precursors according to standard protocols.

Detection of the presence of the target nucleic acid in the test sample then is indicated by the capture of the detection ligand (P-32) onto the solid surface through series of hybridization interactions described above.

The inclusivity behaviors of the oligonucleotide probes were tested in a version of the liquid hybridization format discussed above.

The 16S riboprobes are expected to hybridize to all Chlamydia and non-Chlamydia 16S rRNAs, respectively. Therefore, a positive hybridization signal in this example assay in indicative of the hybridization behavior of the oligonucleotide probe employed in each experiment. Alternatively, these oligonucleotide probes could have been used to capture and detect P-32-labeled Chlamydia 16S rRNAs prepared, for example, by growth of the Chlamydia strains in radioactive phosphorous.

Fifteen serotypes of Chlamydia trachomatis have been tested. Serovar K was obtained from the American Type Culture Collection (ATCC). The rest (serovars A-LGV), were obtained from the Washington Research Foundation.

Briefly, formalin-fixed Elementary Bodies (EBs) of the indicated serovars of Chlamydia trachomatis were lysed in a solution of 1.0 mg/ml proteinase-K (Boehranger Mannheim Corp.) and 1.6% Sarkosine (Sigma) at 65°C for 15 minutes (final volume of 0.07ml). The samples were removed from the incubator and an equal volume of 5.0 M GuSCN (in 0.1M Tris-HCl pH 7.0, 10% dextran sulphate) containing poly-dA tailed capture probe and P-32 labeled detector probe (16S riboprobes appropriate to each oligonucleotide probe being tested) at a concentration of 80 ng/ml and 30 ng/ml (specific activity 1 x 10⁹ cpm/ug) was added.

Hybridization of the probes to the target nucleic acids was allowed to proceed at 37°C for 30 minutes after which time the target complex (dA capture probe target : detector probe ) was captured onto oligo-dT derivatized magnetic beads. This capture was accomplished by adding one tenth of a milliliter (0.1 ml) of a mixture containing 0.0625% (w/v) magnetic beads, 4% BSA, 10 mM EDTA, 0.2% Sarkosine, 0.1M Tris-HCl pH 8.0, and 0.05% Bronopol to the hybridization reaction and incubating the mixture for 5 minutes at 37°C.

Following the capture of the probe/target complexes onto the beads, 0.2 ml of a solution containing 1.0M GuSCN, 10mM EDTA, 0.1M Tris pH 7.0, 0.5% Sarkosine, 0.2% BSA, and 0.1% antifoam was added to the reaction tube, and the tube placed in a magnetic field. The beads (with the attached probe/target complexes) were drawn to the sides of the tube by the magnetic field and the liquid phase (containing the non-hybridized target and detector molecules) was removed from the tube by aspiration.

The captured target complex remaining in the tube was washed in this manner 2 more times.

The separated beads finally were suspended in 0.1 ml of a solution containing 3.25M GuSCN, 65mM EDTA, 0.1M Tris-HCl, pH 7.0, 0.5% Sarkosine and 0.5% BSA and incubated at 37°C for 5 minutes. This treatment releases the target complex from the bead via the "melting" of the dT:dA bond between the bead and the dA-tailed capture probe.

The solution was removed and mixed with a fresh aliquot of beads (0.1 ml) and the target complex was "recaptured" at 37°C for 5 minutes as described above. The wash procedure described above also was repeated.

After the third wash and separation, the beads were resuspended in 0.1 ml of the wash buffer and the beads were filtered onto a nitrocellulose membrane and exposed to x-ray film.

The extent of hybridization of each probe was determined by visual inspection of the exposed x-ray film. The results are shown in Table 2. ++++ indicates a hybridization signal equivalent to that of the "control" Chlamydia trachomatis serovar for which a perfect match between probe and target sequences has been explicitly determined by sequence analysis. +++, ++, + and - represent, respectively, very strong, strong, weak and undetectable hybridization signals.

Hybridization of the probes to approximately 1 x 10⁶ of the non-Chlamydia (negative control) bacteria shown in Table 2 was scored in the same fashion.

### Example 2: Dot blot analysis of probe hybridization behavior.

Dot blot analysis, in accordance with well known procedures, involves immobilizing a nucleic acid or a population of nucleic acids on a filter such as nitrocellulose, nylon, or other derivatized membranes which readily can be obtained commercially, specifically for this purpose. Either DNA or RNA can be easily immobilized on such a filter and subsequently can be probed or tested for hybridization under any of a variety of conditions (i.e., stringencies) with nucleotide sequences or probes of interest. Under stringent conditions, probes whose nucleotide sequences have greater complementarity to the target sequence will exhibit a higher level of hybridization than probes containing less complementarity.

For the experiment shown in Table 3, one tenth of a microgram of purified RNA (Lane et al., 1985, Proceedings of the National Academy of Science, USA 82:6955-6959) from each of the indicated organisms was spotted on nitrocellulose filters. The oligo- nucleotide probes were end-labeled with radioactive phosphorous 32, using standard procedures.

For the oligonucleotide probes described herein, hybridization to rRNA targets at 60°C for 14-16 hours (in a hybridization solution containing 0.9 M NaCl, 0.12 M Tris-HCl, pH 7.8, 6 mM EDTA, 0.1 H KPO4, 0.1% SDS, 0.1% pyrophosphate, 0. 002% ficoll, 0.02% BSA, and 0.002% polyvinylpyrrolidine), followed by three 15 minute post-hybridization washes at 60°C (in 0.03 M NaCl, 0.004 M Tris-HCl, pH 7.8, 0.2 mH EDTA, and 0.1% SDS) to remove unbound probes, would be sufficiently stringent to produce the levels of specificity demonstrated in Table 3.

Following hybridization and washing as described above, the hybridization filters were exposed to X-ray film and the intensity of the signal "scored" visually with respect to control spots of known amount of target material (RNA) as described above (Example 1).

While the description of the invention has been made with reference to detecting rRNA, it will be readily understood that the probes described herein and probes complementary to those described herein also will be useful for the detection of the genes (DNA) which specify the rRNA and, accordingly, such probes are to be deemed equivalents to the described probes and encompassed within the scope of the present invention and the appended claims.

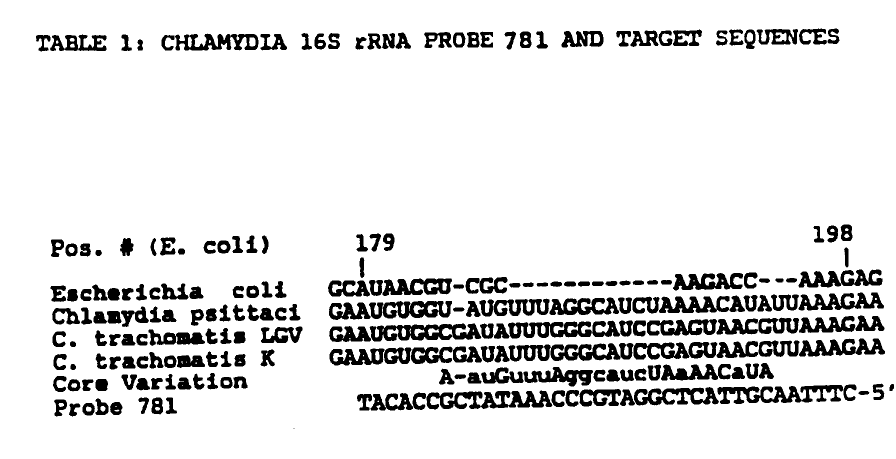

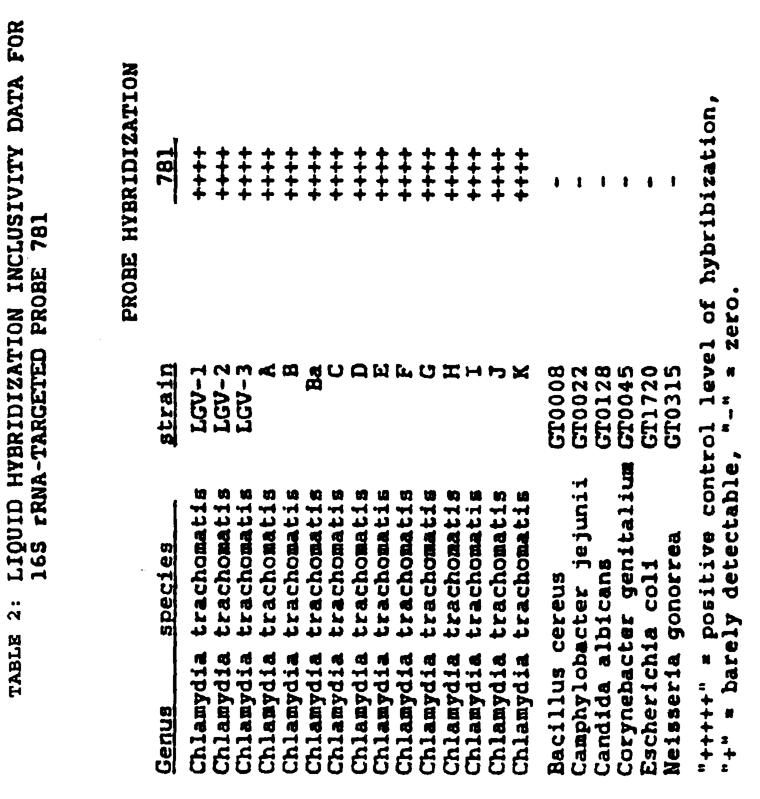

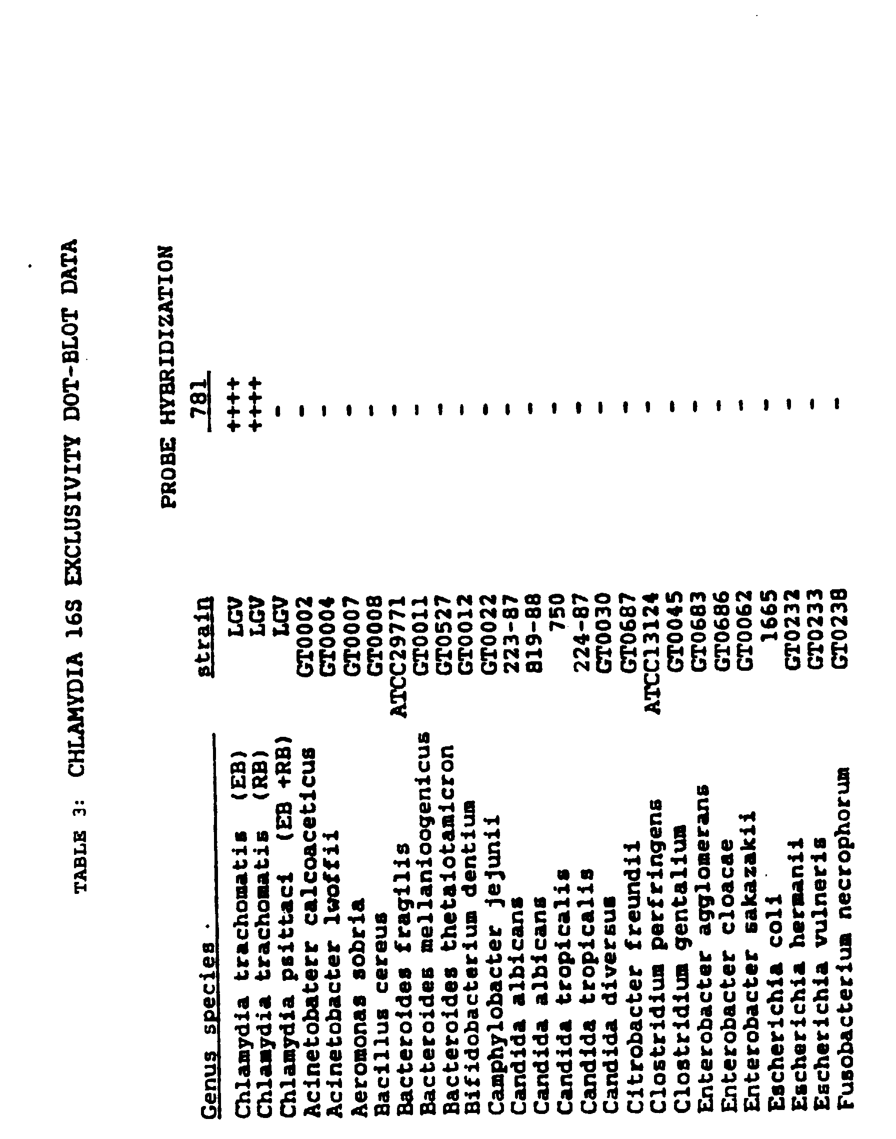

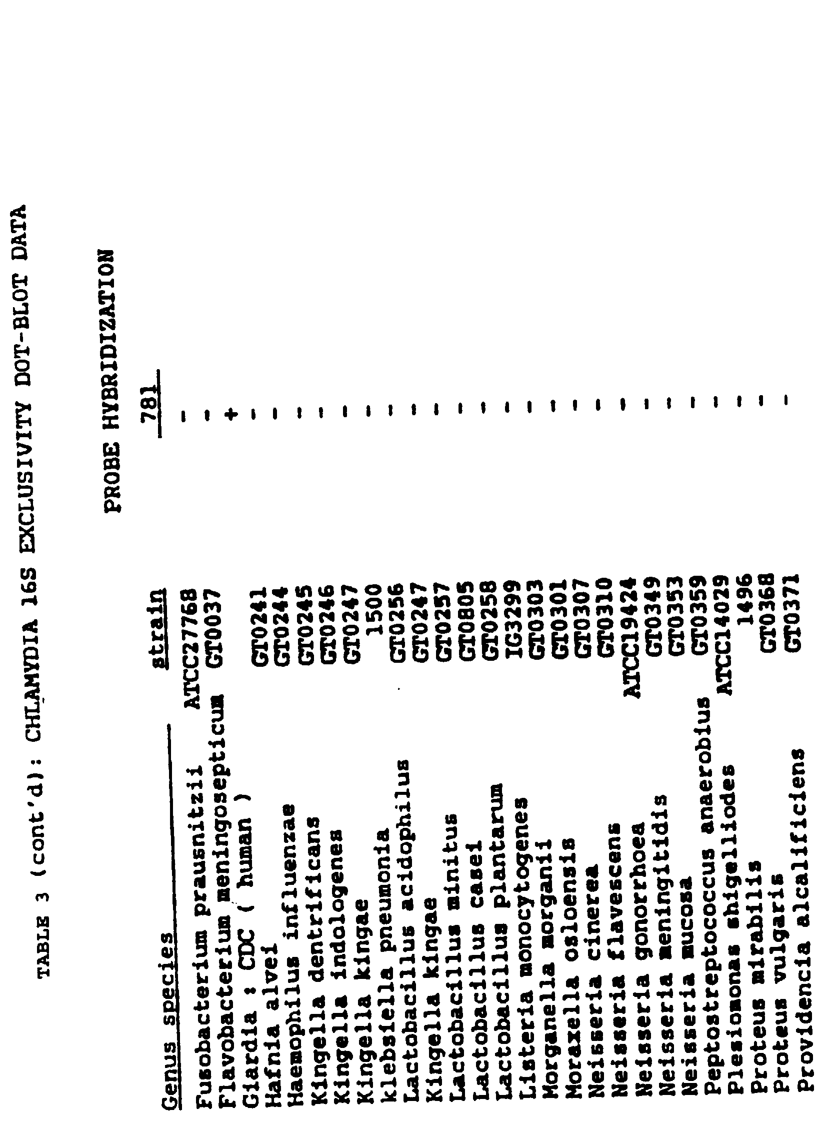

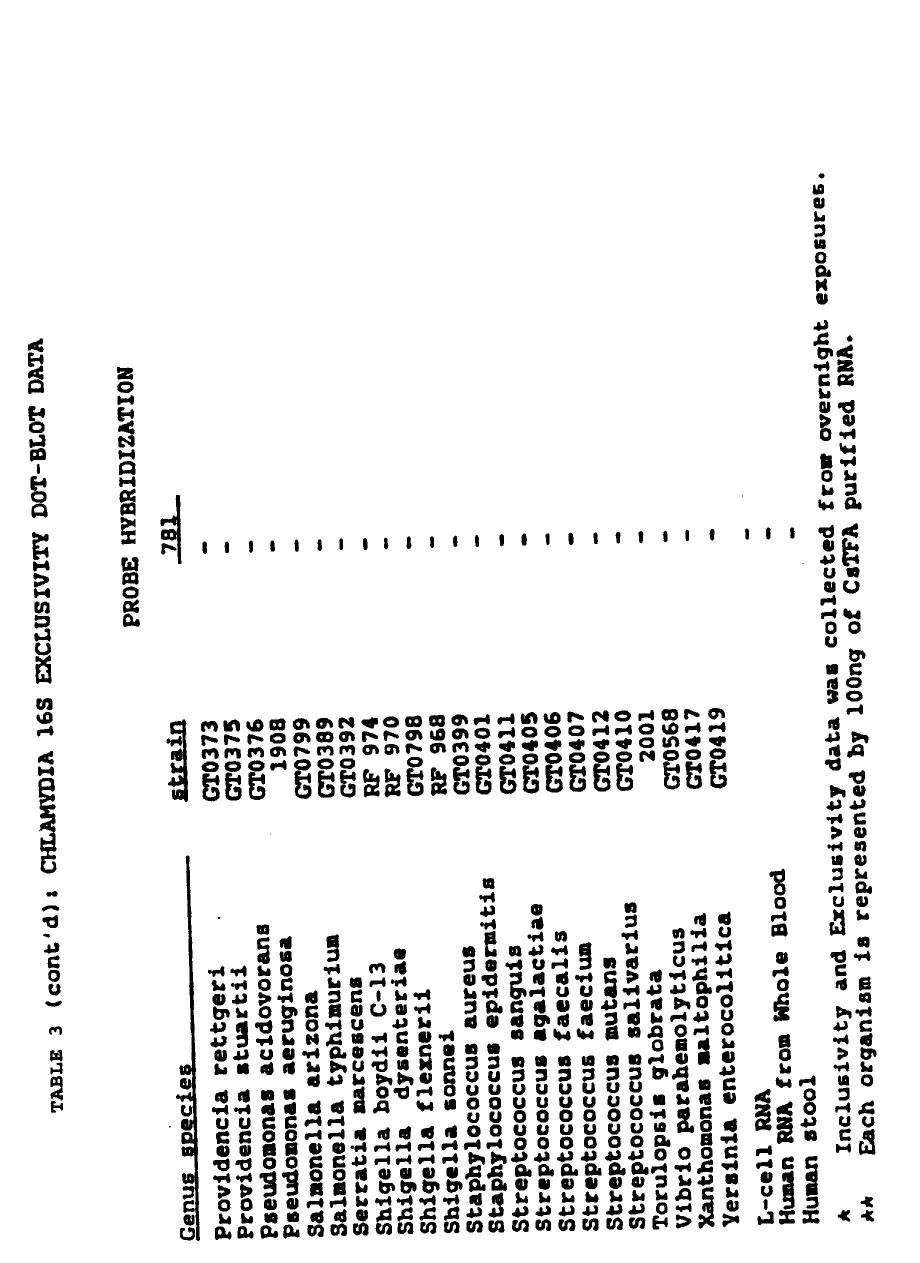

### SEQUENCE LISTINGS

SEQ ID NO: 1
   SEQUENCE LENGTH: 36 bases
   SEQUENCE TYPE: Nucleotide
   STRANDEDNESS: Single
   TOPOLOGY: Linear
   MOLECULE TYPE: DNA probe for Chlamydia trachomatis
   HYPOTHETICAL: No
   SEQUENCE: CTTTAACGTT ACTCGGATGC CCAAATATCG CCACAT 36
SEQ ID NO: 2
   SEQUENCE LENGTH: 35 bases
   SEQUENCE TYPE: Nucleotide
   STRANDEDNESS: Single
   TOPOLOGY: Linear
   MOLECULE TYPE: DNA probe for Chlamydia psittaci
   HYPOTHETICAL: No
   SEQUENCE:

CTTTAATATG TTTTAGATGC CTAAACATAC CACAT 35

## Claims

1. A nucleic acid probe complementary or homologous to at least 90% of a sequence comprising any ten consecutive nucleotides of the region 179 to 198 of the 16S rRNA of either *Chlamydia trachomatis* or *Chlamydia psittaci* (using the *E. coli* numbering system).

2. A probe as claimed in claim 1 which is probe 781 having the sequence: 5'-CTTAACGTTACTCGGATGCCCAAATATCGCCACAT-3'; or probe 782 having the sequence: 5'-CTTTAATATGTTTTAGATGCCTAAACATACCACAT-3'; or one of their complementary sequences.

3. A method of detecting the presence of *Chlamydia trachomatis* in a sample comprising:
a) contacting the sample with at least one probe as claimed in claim 1 specific for *Chlamydia trachomatis;* and
b) detecting a hybrid nucleic acid complex as an indication of the presence of *Chlamydia trachomatis* in the sample.

4. A method as claimed in claim 3, wherein the probe is probe 781 as defined in claim 2.

5. A method of detecting the presence of *Chlamydia psittaci* in a sample comprising:
(a) contacting the sample with at least one probe as claimed in claim 1 specific for *Chlamydia psittaci; and*
(b) detecting a hybrid nucleic acid complex as an indication of the presence of *Chlamydia psittaci* in the sample.

6. A method as claimed in claim 5 wherein the probe is probe 782 as defined in claim 2.

## Patentansprüche

1. Nucleinsäuresonde, die komplementär oder homolog zu mindestens 90 % einer Sequenz ist, die beliebige zehn aufeinanderfolgende Nucleotide des Bereiches 179 bis 198 der 16S-rRNA von Chlamydia trachomatis oder Chlamydia psittaci (unter Verwendung des E. coli-Numerierungssystems) umfaßt.

2. Sonde nach Anspruch 1, nämlich Sonde 781 mit der folgenden Sequenz: 5'-CTTAACGTTACTCGGATGCCCAAATATCGCCACAT-3'; oder Sonde 782 mit der folgenden Sequenz: 5'-CTTTAATATGTTTTAGATGCCTAAACATACCACAT-3'; oder eine ihrer komplementären Sequenzen.

3. Verfahren zum Nachweis der Gegenwart von Chlamydia trachomatis in einer Probe, umfassend:
a) Inkontaktbringen der Probe mit mindestens einer Sonde nach Anspruch 1, die spezifisch für Chlamydia trachomatis ist; und
b) Nachweis eines Hybridnucleinsäurekomplexes als Anzeichen für die Gegenwart von Chlamydia trachomatis in der Probe.

4. Verfahren nach Anspruch 3, wobei die Sonde die Sonde 781 gemäß der Definition in Anspruch 2 ist.

5. Verfahren zum Nachweis der Gegenwart von Chlamydia psittaci in einer Probe, umfassend:
(a) Inkontaktbringen der Probe mit mindestens einer Sonde nach Anspruch 1, die spezifisch für Chlamydia psittaci ist; und
(b) Nachweis eines Hybridnucleinsäurekomplexes als Anzeichen für die Gegenwart von Chlamydia psittaci in der Probe.

6. Verfahren nach Anspruch 5, wobei die Sonde die Sonde 782 gemäß der Definition in Anspruch 2 ist.

## Revendications

1. Sonde d'acide nucléique complémentaire ou homologue d'au moins 90% d'une séquence comprenant n'importe quelle dizaine de nucléotides consécutifs de la région 179 à 198 de 1' ARNr 16S ou bien de *Chlamydia trachomatis* ou bien de *Chlamydia psittaci* (en utilisant le système de numérotation d'*E. coli*).

2. Sonde selon la revendication 1, qui est la sonde 781 ayant la séquence : 5'-CTTAACGTTACTCGGATGCCCAAATATCGCCACAT-3' ; ou la sonde 782 ayant la séquence : 5'-CTTTAATATGTTTTAGATGCCTAAACATACCACAT-3' ; ou l'une de leurs séquences complémentaires.

3. Procédé de détection de la présence de *Chlamydia trachomatis* dans un échantillon, comprenant :
(a) la mise en contact de l'échantillon avec au moins une sonde telle que définie à la revendication 1, spécifique pour *Chlamydia trachomatis* ; et
(b) la détection d'un complexe d'acide nucléique hybride en tant qu'indication de la présence de *Chlamydia trachomatis* dans l'échantillon.

4. Procédé selon la revendication 3, dans lequel la sonde est la sonde 781 telle que définie à la revendication 2.

5. Procédé de détection de la présence de *Chlamydia psittaci* dans un échantillon, comprenant :
(a) la mise en contact de l'échantillon avec au moins une sonde telle que définie à la revendication 1, spécifique pour *Chlamydia psittaci* ; et
(b) la détection d'un complexe d'acide nucléique hybride en tant qu'indication de la présence de *Chlamydia psittaci* dans l'échantillon.

6. Procédé selon la revendication 5, dans lequel la sonde est la sonde 782 telle que définie à la revendication 2.
